# EUROPÄISCHE PATENTSCHRIFT

(11) **EP 3 319 657 B1**
(45) Veröffentlichungstag und Bekanntmachung des Hinweises auf die Patenterteilung: **16.09.2020**
(21) Anmeldenummer: 16741230.3
(22) Anmeldetag: 06.07.2016
(51) Int. Cl.: A61M 1/16

(54) **VORRICHTUNG UND VERFAHREN ZUR DESINFEKTION EINES HYDRAULISCHEN KREISLAUFES**
DEVICE AND METHOD FOR DISINFECTING A HYDRAULIC CIRCUIT
DISPOSITIF ET PROCÉDÉ DE DÉSINFECTION D'UN CIRCUIT HYDRAULIQUE

(30) Priorität: 07.07.2015 DE 102015008783
(43) Veröffentlichungstag der Anmeldung: 16.05.2018
(73) Patentinhaber: Fresenius Medical Care Deutschland GmbH, 61352 Bad Homburg (DE)
(72) Erfinder: GLASER, Benedict, 97421 Schweinfurt (DE); INTERWIES, Steffen, 97422 Schweinfurt (DE); BRANDL, Matthias, 97631 Bad Königshofen (DE)
(74) Vertreter: Herrmann, Uwe
(86) Internationale Anmeldenummer: PCT/EP2016/001163
(87) Internationale Veröffentlichungsnummer: WO 2017/005366

(56) Entgegenhaltungen:
- EP-A1- 1 514 563
- WO-A1-2015/101508
- CN-A- 104 740 707
- US-A- 5 244 568

## Beschreibung

Die vorliegende Erfindung betrifft eine Vorrichtung zur Desinfektion eines hydraulischen Kreislaufes eines Gerätes zur Verarbeitung von Flüssigkeiten für die Dialyse umfassend eine Ventilanordnung.

Eine derartige Vorrichtung ist aus dem Stand der Technik bekannt. Dabei kann die Vorrichtung eingesetzt werden, um den hydraulischen Kreislauf beispielsweise einer Wasserentnahmestation zu desinfizieren, die zur Bereitstellung von aufbereitetem und nach dem Umkehrosmosebetrieb gereinigtem Wasser dient. Diese Füllstation kann die erforderliche Menge gereinigten Wassers an einen mobilen Tank abgeben.

Aus der DE 10 2010 011 465 A1 ist eine Füllstation bekannt, die zur Abgabe von Dialysierflüssigkeit an einen mobilen Tank dient. Dieser Tank wird zu dem Behandlungsplatz verfahren und dann mit dem Dialysegerät verbunden. Das Dialysegerät bezieht die zur Behandlung erforderliche Dialysierflüssigkeit aus dem mobilen Tank.

Aus der WO 2008/104367 A2 ist eine mobile Anordnung bekannt, die zur Herstellung der gebrauchsfertigen Dialyselösung dient.

Hydraulische Kreisläufe von Dialysegeräten oder anderen Komponenten, wie beispielsweise der o.g. Füllstation müssen regelmäßig desinfiziert werden. Dies erfolgt beispielsweis dadurch, dass in den hydraulischen Kreislauf ein im Allgemeinen flüssiges Desinfektionsmittel durch Unterdruckerzeugung im hydraulischen Kreislauf eingezogen wird.

Um diesen Prozess automatisieren zu können und nicht für jede Desinfektion erneut das Desinfektionsmittel an den hydraulischen Kreislauf anschließen zu müssen, ist es notwendig, eine größere Menge an Desinfektionsmittel in einem Behälter direkt an die Gerätehydraulik bzw. an den hydraulischen Kreislauf anzuschließen. Bekannt ist es beispielsweise, einen Kanister mit einem Füllvolumen von 5 bis 10 I mit flüssigem Desinfektionsmittel mit dem hydraulischen Kreislauf zu verbinden.

Damit ist zwar der Vorteil eines einfachen Handlings verbunden, allerdings besteht ein Nachteil darin, dass bei einem Unterdruck im hydraulischen Kreislauf durch eine Leckage an einem Ventil, das den hydraulischen Kreislauf von der Quelle für das Desinfektionsmittel trennt, oder durch eine fehlerhafte Ventilansteuerung Desinfektionsmittel in den hydraulischen Kreislauf gelangen kann, was unter Umständen zu einer Schädigung des Patienten führt.

Ein weiterer Nachteil besteht darin, dass das Desinfektionsmittel im Ansaugschlauch oder in den Ventilen auskristallisieren kann, da zwischen den einzelnen Desinfektionszyklen im Allgemeinen mehrere Tage bis Wochen liegen können. Ein solches Auskristallisieren kann zu einer Störung bzw. zu einem Ausfall der betreffenden Komponente, wie beispielsweise zu einem nicht korrekt schließenden Ventil führen.

Um mit großer Wahrscheinlichkeit das unerwünschte Ansaugen von Desinfektionsmittel in den hydraulischen Kreislauf zu verhindern, werden bei bekannten Geräten zwei in Serie geschaltete Ventile verwendet. Des Weiteren ist es bekannt, nur ein Ventil einzusetzen, dieses jedoch einem regelmäßigen Dichtigkeitstest zu unterziehen, was mit einem zusätzlichen Aufwand verbunden ist. Aus der EP 0 436 855 A1 ist ein Desinfektionsverfahren bekannt, bei dem das Dialysegerät über Stichleitungen einerseits mit einer absperrbaren Frischwasserzulaufleitung und andererseits mit einer absperrbaren Wasserablaufleitung verbunden ist. Zwischen diesen Leitungen befindet sich eine Verbindungsleitung, die durch ein Ventil absperrbar ist. In einem ersten Schritt wird das Dialysegerät bei geschlossenem Ventil der Verbindungsleitung mit Frischwasser durchspült und in einem zweiten Schritt wird bei geöffnetem Ventil und abgesperrter Frischwasserzulaufleitung und abgesperrter Wasserablaufleitung erwärmtes Wasser zirkuliert.

Die EP 1 514 563 A1 beschreibt eine Anordnung zur Desinfektion eines Dialysegerätes, bei der zwischen der Quelle für das Desinfektionsmittel und dem hydraulischen Kreislauf des Dialysegerätes ein Aufnahmeraum angeordnet ist. Über eine siphonartige Verbindung des Aufnahmeraums mit der Quelle für das Desinfektionsmittel wird verhindert, dass z.B. bei einem defekten Ventil Flüssigkeit aus dem Dialysatkreislauf in die Quelle für das Desinfektionsmittel gelangt.

US5224568 A offenbart einen weiteren relevanten Stand der Technik.

Der vorliegenden Erfindung liegt die Aufgabe zugrunde, ein Desinfektionsverfahren und eine Vorrichtung zur Desinfektion dahingehend weiterzubilden, dass mit vergleichsweise geringem Aufwand verhindert werden kann, dass Desinfektionsmittel unbeabsichtigt in den hydraulischen Kreislauf des Gerätes, wie beispielsweise einer Füllstation oder eines Dialysegerätes gelangt.

Diese Aufgabe wird durch eine Vorrichtung mit den Merkmalen des Anspruchs 1 gelöst.

Danach ist vorgesehen, dass die Vorrichtung unter anderem folgendes umfasst:
- wenigstens eine Ventilanordnung;
- wenigstens eine Quelle für Desinfektionsmittel;
- wenigstens eine erste Leitung, die mit der Quelle für Desinfektionsmittel sowie mit dem hydraulischen Kreislauf in Verbindung steht oder verbindbar ist;
- wenigstens eine zweite Leitung, die von der ersten Leitung abzweigt und mit Umgebungsluft oder mit einem Luftreservoir in Verbindung steht;
- wobei die Ventilanordnung wenigstens ein erstes und wenigstens ein zweites Ventil aufweist, die sich in der ersten Leitung vor und nach dem Punkt der Abzweigung der zweiten Leitung von der ersten Leitung befinden, und wobei die Ventilanordnung des Weiteren wenigstens ein drittes Ventil aufweist, das sich in der zweiten Leitung befindet,
- wobei die erste und die zweite Leitung derart angeordnet sind, dass bei geöffnetem ersten und dritten Ventil die zweite Leitung sowie die erste Leitung zwischen dem zweiten Ventil und der Quelle belüftet sind, so dass das darin befindliche Desinfektionsmittel zur Quelle hin abläuft.

Durch diese Vorrichtung wird sichergestellt, dass bei geschlossenem zweiten Ventil, d.h. bei einer Trennung der erfindungsgemäßen Vorrichtung von dem hydraulischen Kreislauf des Gerätes, selbst dann kein Desinfektionsmittel in den hydraulischen Kreislauf gelangt, wenn das zweite Ventil nicht dicht schließen sollte oder versehentlich offensteht. Dies wird dadurch verhindert, dass die erste und die zweite Leitung so angeordnet sind, dass das Desinfektionsmittel bei geschlossenem zweiten Ventil zu der Quelle zurückläuft und nicht in der ersten und zweiten Leitung stehen bleibt, wenn keine Desinfektion durchgeführt wird.

Sollte das zweite Ventil nicht dicht schließen oder versehentlich offen stehen, wird bei in dem hydraulischen Kreislauf herrschenden Unterdruck, der vorzugsweise durch eine in diesem befindliche Pumpe erzeugt wird, Luft und kein Desinfektionsmittel in den hydraulischen Kreislauf eingezogen. Eine unbeabsichtigte Beaufschlagung mit Desinfektionsmittel wird somit sicher verhindert.

Dazu ist keine Serienschaltung zweier Absperrventile notwendig, die die Vorrichtung von dem Gerät trennen, wodurch sich eine entsprechende apparative Vereinfachung ergibt.

An dieser Stelle wird darauf hingewiesen, dass der Begriff "Luft" jedes beliebige Gas oder Gasgemisch umfasst. Der Begriff der "Quelle" für das Desinfektionsmittel umfasst jedes beliebige Einheit, mittels derer Desinfektionsmittel bereitgestellt werden kann, wie z.B. einen Behälter, Beutel, eine Leitung etc.

Eine Steuer- oder Regelungseinheit Z ist vorhanden, die derart ausgebildet ist, dass sie die Ventilanordnung in wenigstens einem ersten Betriebsmodus, in dem dem hydraulischen Kreislauf kein Desinfektionsmittel zugeführt wird, und in wenigstens einem zweiten Betriebsmodus, in dem dem hydraulischen Kreislauf Desinfektionsmittel zugeführt wird, betreibt, wobei in dem ersten Betriebsmodus das zweite Ventil und vorzugsweise auch das erste Ventil geschlossen sind und das dritte Ventil geöffnet ist und wobei in dem zweiten Betriebsmodus das erste und das zweite Ventil geöffnet und das dritte Ventil geschlossen ist.

Die Steuer- oder Regelungsvorrichtung ist derart ausgebildet, dass diese nach Abschluss des zweiten Betriebsmodus, d.h. nach der Zufuhr von Desinfektionsmittel zu dem hydraulischen Kreislauf, die Ventilanordnung so betreibt, dass zunächst das zweite Ventil geschlossen und das dritte Ventil geöffnet wird und dass anschließend das erste Ventil geschlossen wird.

Die vorliegende Erfindung betrifft die Vorrichtung zur Desinfektion eines Gerätes mit einem hydraulischen Kreislauf als solche sowie auch die Kombination der Vorrichtung mit einem derartigen Gerät, d.h. z.B. die Kombination der Vorrichtung mit einer Füllstation oder einem Dialysegerät.

Die Vorrichtung kann fest oder lösbar mit dem Gerät verbunden sein.

In dem hydraulischen Kreislauf des Gerätes befindet sich wenigstens eine Pumpe zur Erzeugung von Unterdruck in dem hydraulischen Kreislauf, wobei die erste Leitung auf der Saugseite der Pumpe in den hydraulischen Kreislauf mündet. Diese Pumpe wird z.B. zur Rezirkulation einer Dialysierflüssigkeit oder auch des Desinfektionsmittels eingesetzt.

In dem hydraulischen Kreislauf befindet sich wenigstens ein viertes Ventil, das in Strömungsrichtung der Flüssigkeit in dem hydraulischen Kreislauf stromaufwärts der Einmündung der ersten Leitung in den hydraulischen Kreislauf angeordnet ist. Vorzugswiese mündet die erste Leitung zwischen diesem vierten Ventil und der Pumpe des hydraulischen Kreislaufes in diesen ein.

Die Steuer- oder Regelungseinheit kann derart ausgebildet sein, dass diese in dem ersten Betriebsmodus (d.h. wenn kein Desinfektionsmittel zugeführt wird) bewirkt, dass das vierte Ventil geöffnet ist und in dem zweiten Betriebsmodus (d.h. wenn Desinfektionsmittel zugeführt wird) bewirkt, dass das vierte Ventil geschlossen ist.

Bei den Ventilen handelt es sich vorzugsweise um Auf-/Zuventile, die in genau zwei Positionen stehen können, d.h. in der Schließstellung und in der geöffneten Position. Von der Erfindung ist jedoch auch der Fall umfasst, dass es sich bei den Ventilen um Drosselventile handelt, deren Strömungsquerschnitt zwischen der Offen- und der Schließposition z.B. stufenlos veränderbar ist.

Bei den Ventilen kann es sich um manuell zu betätigende Ventile handeln, bevorzugt ist es jedoch, wenn es sich um mit einem Antrieb versehene Ventile handelt, die von einer Steuer- oder Regelungseinheit angesteuert werden können. Erfindungsgemäß handelt es sich bei dem ersten, zweiten und dritten Ventil um mit einen Antrieb versehene Ventile, die von einer Steuer- oder Regelungseinheit angesteuert werden können.

Vorzugsweise handelt es bei dem ersten, zweiten und dritten Ventil um elektrisch betriebene Ventile.

Vorzugsweise ist das erste und das zweite Ventil stromlos geschlossen und das dritte Ventil stromlos offen.

Bei dem hydraulischen Kreislauf kann es sich um einen hydraulischen Kreislauf einer Füllstation für die Befüllung eines Behälters oder eines Dialysegerätes mit Dialyseflüssigkeit, RO-Wasser oder einer sonstigen Flüssigkeit handeln oder um einen hydraulischen Kreislauf eines Dialysegerätes oder eines sonstigen Gerätes zur Blutbehandlung.

Die vorliegende Erfindung betrifft des Weiteren ein Verfahren zur Desinfektion eines hydraulischen Kreislaufes eines Gerätes zur Verarbeitung von Flüssigkeiten für die Dialyse, wobei in einem ersten Betriebsmodus dem hydraulischen Kreislauf kein Desinfektionsmittel zugeführt wird und wobei in einem zweiten Betriebsmodus dem hydraulischen Kreislauf Desinfektionsmittel zugeführt wird, wobei zur Desinfektion des hydraulischen Kreislaufs zumindest eine Vorrichtung nach einem der Ansprüche 1 bis 4 genutzt wird, die wenigstens eine Quelle für das Desinfektionsmittel aufweist sowie wenigstens eine erste Leitung, die sich von der Quelle für das Desinfektionsmittel zu dem hydraulischen Kreislauf erstreckt und in der sich wenigstens ein Ventil zum Absperren der ersten Leitung befindet, wobei in dem ersten Betriebsmodus die erste Leitung geschlossen ist und zwischen dem Ventil und der Quelle belüftet wird und wobei in dem zweiten Betriebsmodus die erste Leitung geöffnet ist und nicht belüftet wird..

Denkbar ist es, dass in dem zweiten Betriebsmodus an der Stelle in dem hydraulischen Kreislauf Unterdruck erzeugt wird, an der die erste Leitung in den hydraulischen Kreislauf mündet.

Weiterhin kann vorgesehen sein, dass der hydraulische Kreislauf derart betrieben wird, dass in dem zweiten Betriebsmodus ein Ventil geschlossen wird, das sich stromaufwärts der Stelle in dem hydraulischen Kreislauf befindet, an der die erste Leitung in den hydraulischen Kreislauf mündet. In einer weiteren bevorzugten Ausgestaltung befindet sich stromabwärts dieser Einmündung eine Pumpe zur Erzeugung eines Unterdrucks im Bereich der Einmündung.

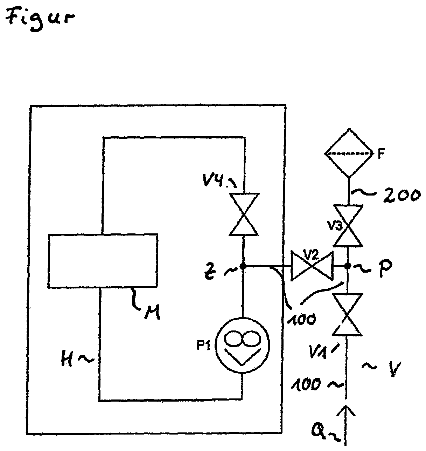

Weitere Einzelheiten und Vorteile der Erfindung werden anhand eines in der Zeichnung dargestellten Ausführungsbeispiels näher erläutert.

Die einzige Figur zeigt ein Gerät mit einem hydraulischen Kreislauf sowie die daran angeschlossene Vorrichtung zu dessen Desinfektion.

Das Bezugszeichen H zeigt das hydraulische System bzw. den hydraulischen Kreislauf einer Füllstation.

In dem hydraulischen Kreislauf H befindet sich die Pumpe P1 sowie ein Ventil V4, das im Rahmen der Erfindung als viertes Ventil bezeichnet wird.

In dem hydraulischen Kreislauf H befindet sich darüber hinaus ein Mischgefäß M, in dem verschiedene, zur Herstellung einer Dialyselösung benötigte Komponenten gemischt werden. Diese Mischung wird mittels der Pumpe P1 rezirkuliert. Soll die Dialyseflüssigkeit beispielsweise an einen mobilen Tank oder an ein Dialysegerät abgegeben werden, erfolgt dies über eine nicht dargestellte Ablaufleitung.

Um den hydraulischen Kreislauf H desinfizieren zu können ist die Vorrichtung V vorgesehen, die eine Quelle Q für das Desinfektionsmittel aufweist sowie eine Ventilanordnung bestehend aus dem ersten Ventil V1, dem zweiten Ventil V2 und dem dritten Ventil V3.

Das Bezugszeichen 100 kennzeichnet eine erste Leitung, die sich von der Quelle Q zu dem hydraulischen Kreislauf H erstreckt und in diesen an der Stelle Z mündet.

Zwischen dem ersten V1 und dem zweiten Ventil V2 zweigt an der Stelle P von der ersten Leitung 100 eine zweite Leitung 200 ab, deren anderes Ende mit der Umgebungsatmosphäre in Verbindung steht. Im Endbereich der zweiten Leitung 200 befindet sich ein Filter F, der das Eindringen von Verschmutzungen in die zweite Leitung 200 von der Umgebung verhindert.

Die Ventile V1, V2 und V4 sind normale Absperrventile, die unbestromt geschlossen sind. Das Ventil V3 ist ein Schlauchquetschventil, welches unbestromt offen ist.

Der Totraum zwischen den Ventilen V1, V2 und V3 sollte sehr klein gehalten werden. Dies gilt entsprechend für das Leitungsstück zwischen dem Ventil V2 und der Strecke zwischen V4 und der Pumpe P1.

Im normalen Betrieb, d.h. in dem erfindungsgemäßen ersten Betriebsmodus, in dem kein Desinfektionsmittel dem hydraulischen Kreislauf H zugeführt wird, sind die Ventile V1, V2 und V3 unbestromt, d.h. V1 und V2 sind geschlossen und V3 ist geöffnet. Die Leitung 200 und der Abschnitt der ersten Leitung 100 von V2 bis zu der Quelle Q sind belüftet, d.h. stehen mit der Umgebungsatmosphäre in Verbindung.

Soll Desinfektionsmittel (zweiter Betriebsmodus gemäß der Erfindung) angesaugt werden, wird V4 geschlossen, V2 und V1 geöffnet und V3 geschlossen. Das Leitungssystem der Vorrichtung V ist somit von der Umgebungsatmosphäre abgetrennt und durch den Unterdruck, der durch die laufende Pumpe P1 erzeugt wird, wird Desinfektionsmittel von der Quelle Q in den hydraulischen Kreislauf H gesogen.

Nach dem erfolgten Ansaugen von Desinfektionsmittel wird zunächst V2 geschlossen und V3 geöffnet. Dies führt dazu, dass das restliche Desinfektionsmittel, das sich in der zweiten Leitung 200 und in dem Abschnitt der ersten Leitung 100 zwischen dem Ventil V2 und der Quelle Q befindet, zurück zu der Quelle fließt. Diese Leitungsabschnitte sind sodann luftgefüllt. Dieses Zurückfließen erfolgt schwerkraftbedingt.

Anschließend wird das Ventil V1 geschlossen.

Durch die hydrostatische Anordnung der Leitungen 100 und 200 sowie der Ventile V1, V2 und V3 ist sichergestellt, dass die Ansaugstrecke zwischen dem ersten Ventil V1 und der Quelle Q vollständig leer läuft. Dies gilt entsprechend für das Leitungsstück von dem zweiten Ventil V2 bis zu dem ersten Ventil V1.

Somit ist sichergestellt, dass nach Abschluss des Desinfektionsvorgangs bzw. im normalen Betrieb des Gerätes an dem Ventil V2 an der vom hydraulischen Kreislauf abgewandten Seite Luft und nicht Desinfektionsmittel anliegt.

Sollte das Ventil V2 falsch angesteuert werden, z.B. während der Behandlung oder während der Zubereitung von Dialysierflüssigkeit offen stehen, oder nicht dicht schließen, wird Luft über das Ventil V3 angesaugt.

Ein versehentliches Eindringen von Desinfektionsmittel in den hydraulischen Kreislauf H kann somit verhindert werden. Eine Ventilanordnung mit zwei in Serie geschalteten Ventilen, wie beispielsweise zwei in Serie geschaltete Ventile V3 zwischen der Vorrichtung und dem hydraulischen Kreislauf ist nicht notwendig und vorzugsweise nicht vorgesehen.

## Patentansprüche

1. Vorrichtung zur Desinfektion eines hydraulischen Kreislaufes (H) eines Gerätes zur Verarbeitung von Flüssigkeiten für die Dialyse, wobei die Vorrichtung folgendes umfasst:
wenigstens eine Ventilanordnung;
wenigstens eine Quelle (Q) für Desinfektionsmittel;
wenigstens eine erste Leitung (100), die mit der Quelle (Q) für Desinfektionsmittel sowie mit dem hydraulischen Kreislauf (H) in Verbindung steht oder verbindbar ist;
wenigstens eine zweite Leitung (200), die von der ersten Leitung (100) abzweigt und mit Umgebungsluft oder mit einem Luftreservoir in Verbindung steht;
wobei die Ventilanordnung wenigstens ein erstes (V1) und wenigstens ein zweites Ventil (V2) aufweist, die sich in der ersten Leitung (100) vor und nach dem Punkt (P) der Abzweigung der zweiten Leitung (200) von der ersten Leitung (100) befinden,
wobei die Ventilanordnung des Weiteren wenigstens ein drittes Ventil (V3) aufweist, das sich in der zweiten Leitung (200) befindet,
wobei die erste (100) und die zweite Leitung (200) derart angeordnet sind, dass bei geöffnetem ersten (V1) und dritten Ventil (V3) die zweite Leitung (200) sowie die erste Leitung (100) zwischen dem zweiten Ventil (V2) und der Quelle (Q) belüftet sind, so dass das darin befindliche Desinfektionsmittel zur Quelle (Q) hin abläuft,
wobei sich in dem hydraulischen Kreislauf (H) wenigstens eine Pumpe (P1) zur Erzeugung von Unterdruck in dem hydraulischen Kreislauf (H) befindet und die erste Leitung (200) auf der Saugseite der Pumpe (P1) in den hydraulischen Kreislauf (H) mündet, und
wobei sich in dem hydraulischen Kreislauf (H) wenigstens ein viertes Ventil (V4) befindet, das in Strömungsrichtung der Flüssigkeit in dem hydraulischen Kreislauf (H) stromaufwärts der Einmündung der ersten Leitung (200) in den hydraulischen Kreislauf (H) angeordnet ist, wobei wenigstens eine Steuer- oder Regelungseinheit vorhanden ist, die derart ausgebildet ist, dass sie die Ventilanordnung in wenigstens einem ersten Betriebsmodus, in dem dem hydraulischen Kreislauf (H) kein Desinfektionsmittel zugeführt wird, und in wenigstens einem zweiten Betriebsmodus, in dem dem hydraulischen Kreislauf (H) Desinfektionsmittel zugeführt wird, betreibt, wobei in dem ersten Betriebsmodus das erste (V1) und das zweite Ventil (V2) geschlossen und das dritte Ventil (V3) geöffnet ist und wobei in dem zweiten Betriebsmodus das erste (V1) und das zweite Ventil (V2) geöffnet und das dritte Ventil (V3) geschlossen ist, **dadurch gekennzeichnet, dass** die Steuer- oder Regelungseinheit derart ausgebildet ist, dass diese nach Abschluss des zweiten Betriebsmodus, d.h. nach der Zufuhr von Desinfektionsmittel zu dem hydraulischen Kreislauf (H), die Ventilanordnung so betreibt, dass zunächst das zweite Ventil (V2) geschlossen und das dritte Ventil (V3) geöffnet wird und dass anschließend das erste Ventil (V1) geschlossen wird.

2. Vorrichtung nach einem der vorhergehenden Ansprüche, **dadurch gekennzeichnet, dass** die Steuer- oder Regelungseinheit derart ausgebildet ist, dass diese in dem ersten Betriebsmodus bewirkt, dass das vierte Ventil (V4) geöffnet ist und in dem zweiten Betriebsmodus bewirkt, dass das vierte Ventil (V4) geschlossen ist.

3. Vorrichtung nach einem der vorhergehenden Ansprüche, **dadurch gekennzeichnet, dass** es sich bei dem ersten (V1) bis dritten Ventil (V3) um elektrisch betriebene Ventile handelt, wobei das erste (V1) und das zweite Ventil (V2) stromlos geschlossen und das dritte Ventil (V3) stromlos offen ist.

4. Vorrichtung nach einem der vorhergehenden Ansprüche, **dadurch gekennzeichnet, dass** es sich bei dem hydraulischen Kreislauf (H) um einen hydraulischen Kreislauf (H) einer Füllstation für die Befüllung eines Behälters oder eines Dialysegerätes mit Dialyseflüssigkeit oder mit einer sonstigen Flüssigkeit oder um einen hydraulischen Kreislauf eines Dialysegerätes handelt.

5. Verfahren zur Desinfektion eines hydraulischen Kreislaufes eines Gerätes zur Verarbeitung von Flüssigkeiten für die Dialyse, wobei in einem ersten Betriebsmodus dem hydraulischen Kreislauf (H) kein Desinfektionsmittel zugeführt wird und wobei in einem zweiten Betriebsmodus dem hydraulischen Kreislauf (H) Desinfektionsmittel zugeführt wird, wobei zur Desinfektion des hydraulischen Kreislaufs (H) zumindest eine Vorrichtung nach einem der vorhergehenden Ansprüche genutzt wird, wobei in dem ersten Betriebsmodus die erste Leitung (100) geschlossen ist und zwischen dem Ventil (V2) und der Quelle belüftet wird und wobei in dem zweiten Betriebsmodus die erste Leitung (100) geöffnet ist und nicht belüftet wird.

6. Verfahren nach Anspruch 5, **dadurch gekennzeichnet, dass** der hydraulische Kreislauf derart betrieben wird, dass in dem zweiten Betriebsmodus an der Stelle in dem hydraulischen Kreislauf (H) Unterdruck erzeugt wird, an der die erste Leitung (100) in den hydraulischen Kreislauf (H) mündet.

7. Verfahren nach Anspruch 6, **dadurch gekennzeichnet, dass** der hydraulische Kreislauf derart betrieben wird, dass in dem zweiten Betriebsmodus ein Ventil (V4) geschlossen wird, das sich stromaufwärts der Stelle in dem hydraulischen Kreislauf (H) befindet, an der die erste Leitung (100) in den hydraulischen Kreislauf mündet.

## Claims

1. Device for disinfecting a hydraulic circuit (H) of an apparatus for processing liquids for dialysis, wherein the device comprises the following:
at least one valve arrangement;
at least one source (Q) for disinfectant;
at least one first line (100), which is or can be connected to the source (Q) for disinfectant and to the hydraulic circuit (H);
at least one second line (200), which branches off from the first line (100) and is connected to the ambient air or to an air reservoir;
wherein the valve arrangement has at least one first (V1) and at least one second valve (V2), which are located in the first line (100) before and after the point (P) at which the second line (200) branches off from the first line (100),
wherein the valve arrangement furthermore has at least one third valve (V3), which is located in the second line (200),
wherein the first (100) and the second line (200) are arranged such that when the first (V1) and third valve (V3) are open, the second line (200) and the first line (100) are ventilated between the second valve (V2) and the source (Q), so that the disinfectant located therein drains off to the source (Q),
wherein at least one pump (P1) is located in the hydraulic circuit (H) for generating a vacuum in the hydraulic circuit (H) and the first line (200) opens on the suction side of the pump (P1) into the hydraulic circuit (H), and
wherein located in the hydraulic circuit (H) is at least one fourth valve (V4), which is arranged in the flow direction of the liquid in the hydraulic circuit (H) upstream of the opening of the first line (200) into the hydraulic circuit (H), wherein at least one control or adjustment unit is present, which is designed so that it operates the valve arrangement in at least a first operating mode in which no disinfectant is supplied to the hydraulic circuit (H), and in at least a second operating mode in which disinfectant is supplied to the hydraulic circuit (H), wherein in the first operating mode the first (V1) and the second valve (V2) are closed and the third valve (V3) is open and wherein in the second operating mode the first (V1) and the second valve (V2) are open and the third valve (V3) is closed, **characterised in that** the control or adjustment unit is designed so that after completion of the second operating mode, i.e. following the supply of disinfectant to the hydraulic circuit (H), it operates the valve arrangement such that first the second valve (V2) is closed and the third valve (V3) is opened, and that then the first valve (V1) is closed.

2. Device according to one of the preceding claims, **characterised in that** the control or adjustment unit is designed so that in the first operating mode it causes the fourth valve (V4) to be opened and in the second operating mode it causes the fourth valve (V4) to be closed.

3. Device according to one of the preceding claims, **characterised in that** the first (V1) to third valve (V3) are electrically operated valves, wherein the first (V1) and the second valve (V2) are closed when currentless and the third valve (V3) is open when currentless.

4. Device according to one of the preceding claims, **characterised in that** the hydraulic circuit (H) is a hydraulic circuit (H) of a filling station for filling a container or a dialysis apparatus with dialysis liquid or with another liquid or is a hydraulic circuit of a dialysis apparatus.

5. Method for disinfecting a hydraulic circuit of an apparatus for processing liquids for dialysis, wherein in a first operating mode, no disinfectant is supplied to the hydraulic circuit (H) and wherein in a second operating mode, disinfectant is supplied to the hydraulic circuit (H), wherein to disinfect the hydraulic circuit (H) at least one device according to one of the preceding claims is used, wherein in the first operating mode the first line (100) is closed and is ventilated between the valve (V2) and the source, and wherein in the second operating mode the first line (100) is open and not ventilated.

6. Method according to claim 5, **characterised in that** the hydraulic circuit is operated such that in the second operating mode, a vacuum is generated in the hydraulic circuit (H) at the point at which the first line (100) opens into the hydraulic circuit (H).

7. Method according to claim 6, **characterised in that** the hydraulic circuit is operated such that in the second operating mode, a valve (V4) is closed, which valve is located upstream of the point in the hydraulic circuit (H) at which the first line (100) opens into the hydraulic circuit.

## Revendications

1. Dispositif de désinfection d'un circuit (H) hydraulique d'un appareil servant à traiter des liquides pour la dialyse, dans lequel le dispositif comprend ce qui suit :
au moins un ensemble de soupapes ;
au moins une source (Q) pour des produits de désinfection ;
au moins un premier conduit (100), qui est relié ou peut être relié à la source (Q) pour des produits de désinfection ainsi qu'au circuit (H) hydraulique ;
au moins un deuxième conduit (200), qui forme un embranchement avec le premier conduit (100) et est relié à l'air extérieur ou à un réservoir d'air ;
dans lequel l'ensemble de soupapes présente au moins une première soupape (V1) et au moins une deuxième soupape (V2), qui se trouvent dans le premier conduit (100) avant et après le point (P) de l'embranchement du deuxième conduit (200) par rapport au premier conduit (100),
dans lequel l'ensemble de soupapes présente par ailleurs au moins une troisième soupape (V3), qui se trouve dans le deuxième conduit (200),
dans lequel le premier (100) et le deuxième conduit (200) sont disposés de telle manière que lorsque la première (V1) et la troisième soupape (V3) sont ouvertes, le deuxième conduit (200) ainsi que le premier conduit (100) sont aérés entre la deuxième soupape (V2) et la source (Q) de sorte que le produit de désinfection s'y trouvant s'évacue en direction de la source (Q),
dans lequel au moins une pompe (P1) servant à générer du vide dans le circuit (H) hydraulique se trouve dans le circuit (H) hydraulique et le premier conduit (200) débouche dans le circuit (H) hydraulique sur le côté aspiration de la pompe (P1), et
dans lequel se trouve dans le circuit (H) hydraulique au moins une quatrième soupape (V4), qui est disposée dans la direction d'écoulement du liquide dans le circuit (H) hydraulique en amont de l'embouchure du premier conduit (200) dans le circuit (H) hydraulique, dans lequel est présente au moins une unité de commande ou de régulation, qui est réalisée de telle manière qu'elle fait fonctionner l'ensemble de soupapes dans au moins un premier mode de fonctionnement, dans lequel aucun produit de désinfection n'est amené au circuit (H) hydraulique, et dans au moins un deuxième mode de fonctionnement, dans lequel du produit de désinfection est amené au circuit (H) hydraulique, dans lequel la première (V1) et la deuxième soupape (V2) sont fermées et la troisième soupape (V3) est ouverte dans le premier mode de fonctionnement et dans lequel la première (V1) et la deuxième soupape (V2) sont ouvertes et la troisième soupape (V3) est fermée dans le deuxième mode de fonctionnement, **caractérisé en ce que** l'unité de commande ou de régulation est réalisée de telle manière que celle-ci fait fonctionner l'ensemble de soupapes à l'issue du deuxième mode de fonctionnement, en d'autres termes après l'amenée de produit de désinfection au circuit (H) hydraulique de telle sorte que dans un premier temps la deuxième soupape (V2) est fermée et la troisième soupape (V3) est ouverte et qu'ensuite la première soupape (V1) est fermée.

2. Dispositif selon l'une quelconque des revendications précédentes, **caractérisé en ce que** l'unité de commande ou de régulation est réalisée de telle manière qu'elle a pour effet dans le premier mode de fonctionnement que la quatrième soupape (V4) est ouverte et a pour effet dans le deuxième mode de fonctionnement que la quatrième soupape (V4) est fermée.

3. Dispositif selon l'une quelconque des revendications précédentes, **caractérisé en ce que** la première (V1) à la troisième soupape (V3) sont des soupapes à fonctionnement électrique, dans lequel la première (V1) et la deuxième soupape (V2) sont fermées sans courant et la troisième soupape (V3) est ouverte sans courant.

4. Dispositif selon l'une quelconque des revendications précédentes, **caractérisé en ce que** le circuit ((H) hydraulique est un circuit (H) hydraulique d'une station de remplissage pour le remplissage d'un contenant ou d'un appareil de dialyse avec du liquide de dialyse ou avec un autre liquide ou est un circuit hydraulique d'un appareil de dialyse.

5. Procédé de désinfection d'un circuit hydraulique d'un appareil servant à traiter des liquides pour la dialyse, dans lequel aucun produit de désinfection n'est amené au circuit (H) hydraulique dans un premier mode de fonctionnement et dans lequel du produit de désinfection est amené au circuit hydraulique (H) dans un deuxième mode de fonctionnement, dans lequel au moins un dispositif selon l'une quelconque des revendications précédentes, est utilisé pour désinfecter le circuit (H) hydraulique, dans lequel le premier conduit (100) est fermé et est aéré entre la soupape (V2) et la source dans le premier mode de fonctionnement et dans lequel le premier conduit (100) est ouvert et n'est pas aéré dans le deuxième mode de fonctionnement.

6. Procédé selon la revendication 5, **caractérisé en ce que** le circuit hydraulique fonctionne de telle manière qu'est généré, dans le deuxième mode de fonctionnement, un vide à l'emplacement dans le circuit (H) hydraulique, au niveau duquel le premier conduit (100) débouche dans le circuit (H) hydraulique.

7. Procédé selon la revendication 6, **caractérisé en ce que** le circuit hydraulique fonctionne de telle manière qu'est fermée dans le deuxième mode de fonctionnement une soupape (V4), laquelle se trouve en amont de l'emplacement dans le circuit (H) hydraulique, au niveau duquel le premier conduit (100) débouche dans le circuit hydraulique.
